# EUROPEAN PATENT APPLICATION

(11) **EP 4 470 527 A1**
(43) Date of publication of application: **04.12.2024**
(21) Application number: 22922058.7
(22) Date of filing: 17.11.2022
(51) Int. Cl.: A61K 9/107, A61K 8/36, A01N 25/04, A01N 37/06, A01N 37/10, A01N 43/16, A01N 47/44, A23D 7/00, A23L 29/10, A23L 33/10, A23L 33/115, A61K 8/06, A61K 8/39, A61K 31/122, A61K 31/575, A61K 47/10, A61K 47/12, A61K 47/22, A61K 47/26, A61K 47/34, A61K 47/44

(54) **NANOEMULSION ENDOWED WITH ANTISEPTIC EFFECT**

(30) Priority: 24.01.2022 JP 2022008676
(71) Applicant: MORESCO Corporation, Kobe-shi, Hyogo 650-0047 (JP)
(72) Inventor: KINOSHITA, Yusuke, Kobe-shi, Hyogo 650-0047 (JP); MARUYAMA, Shingo, Kobe-shi, Hyogo 650-0047 (JP)
(74) Representative: Bittner, Thomas L.
(86) International application number: PCT/JP2022/042729
(87) International publication number: WO 2023/139907

(57) **Abstract**

Provided is a nanoemulsion which has stability and to which preservative efficacy is imparted, in a bacterial contamination environment. This object is attained by a nanoemulsion which has an average particle diameter of 30 nm or less, has a particle size distribution index of 0.14 or less, does not contain particles of 100 nm or more, and contains, as a preservative, at least one selected from an organic acid (salt) and a biguanide-based compound.

## Description

### Technical Field

The present invention relates to a nanoemulsion to which preservative efficacy is imparted.

### Background

Emulsification (formation of an emulsion) is known as a method for solubilizing a poorly water-soluble substance which is used in pharmaceutical products, cosmetic products, foods, and the like. The oral and percutaneous absorption efficiency of a poorly water-soluble substance is improved by undergoing an emulsification step. In particular, the effect is greater in a nanoemulsion containing of emulsion particles having a small particle diameter.

Until now, the inventors of the present invention have developed a technique of providing a microemulsion containing emulsified particles having a small particle diameter (see, for example, Patent Literature 1).

### Citation List

### [Patent Literature]

[Patent Literature 1]
   International Publication No. WO 2021/005676
[Patent Literature 2]
   Japanese Patent Application Publication Tokukai No. 2020-183427

### Summary of Invention

### Technical Problem

However, the above-described technique has room for further improvement in terms of preservation from decay.

An object of an aspect of the present invention is to provide a nanoemulsion which has stability and in which decay by bacterial contamination is inhibited.

### Solution to Problem

A nanoemulsion in accordance with an embodiment of the present invention is characterized by containing emulsion particles which have an average particle diameter of 30 nm or less and have a particle size distribution index of 0.14 or less, and not containing emulsion particles of 100 nm or more, the nanoemulsion containing a surfactant component, an oil, a poorly water-soluble functional component, an aqueous medium, and a preservative, the preservative containing at least one selected from an organic acid (salt) and a biguanide-based compound.

### Advantageous Effects of Invention

According to an aspect of the present invention, it is possible to provide a nanoemulsion which has stability and in which decay by bacterial contamination is inhibited.

### Description of Embodiments

The inventors of the present invention have attempted to solve the above problem by adding a preservative to a nanoemulsion which is developed by the inventors of the present invention and in which the particle diameter of emulsified particles is at the level of nanometers. Preservatives such as parabens and alkanediol (propylene glycol) are commonly used in emulsions (see Patent Literature 2 and the like). However, in a case where those preservatives were added to a nanoemulsion, a problem was found in which the stability of the emulsion could not be maintained. This seemed to be due to the very small particle diameter of the emulsified particles in the nanoemulsion. In view of this, the inventors of the present invention have carried out diligent studies, and as a result, the inventors of the present invention have found that the above problem can be solved by using a specific preservative, and thus have accomplished the present invention.

That is, a nanoemulsion in accordance with an embodiment of the present invention contains emulsion particles which have an average particle diameter of 30 nm or less and have a particle size distribution index of 0.14 or less, and does not contain emulsion particles of 100 nm or more, the nanoemulsion containing a surfactant component, an oil, a poorly water-soluble functional component, an aqueous medium, and a preservative, the preservative containing at least one selected from an organic acid (salt) and a biguanide-based compound.

The following will describe embodiments of the present invention in detail. Note, however, that the present invention is not limited to the embodiments. The present invention can be carried out in specific forms into which various modifications are incorporated within the scope set forth herein. All of the academic documents and patent literatures listed herein are incorporated by reference herein. Unless otherwise specified herein, "A to B" which indicates a numerical range means "not less than A and not more than B".

### [1. Nanoemulsion]

In the present specification, the "nanoemulsion" refers to an optically transparent or semitransparent dispersion system of minute droplets that is made of water, oil, and a surfactant and that has a particle diameter of approximately several nanometers to several tens of nanometers. The minute droplets correspond to micelles described later and are emulsion particles (emulsified particles) of a nanoemulsion. A nanoemulsion in accordance with an embodiment of the present invention further contains, in the dispersion system of minute droplets, a poorly water-soluble functional component and a preservative. That is, the nanoemulsion in accordance with an embodiment of the present invention contains a surfactant component, an oil, a poorly water-soluble functional component, an aqueous medium, and a preservative.

The nanoemulsion in accordance with an embodiment of the present invention has an average particle diameter of 30 nm or less, has a particle size distribution index of 0.14 or less, does not contain particles of 100 nm or more, and a preservative contains at least one selected from an organic acid (salt) and a biguanide-based compound.

### (Surfactant component)

The nanoemulsion in accordance with an embodiment of the present specification contains a surfactant component. Examples of the surfactant component preferably include, but not limited to, nonionic surfactants such as polyoxyalkylene alkyl ether; polyoxyalkylene alkenyl ether; polyoxyalkylene alkyl phenyl ether; polyoxyethylene castor oil; sorbitan fatty acid ester and an alkylene oxide adduct thereof; polyoxyalkylene fatty acid ester; polyoxyethylene hydrogenated castor oil; and polyoxyethylene hydrogenated castor oil alkanoate.

More preferably, the nanoemulsion contains, as a main component of the surfactant component, at least one selected from the nonionic surfactants described above. Note here that, in the present specification, the "main component" is preferably not less than 80% by weight, more preferably not less than 85% by weight, even more preferably not less than 90% by weight, particularly preferably not less than 95% by weight, and most preferably 100% by weight, relative to the total weight of the surfactant component contained in the nanoemulsion. Even more preferably, the nanoemulsion contains, as a main component of the surfactant component, at least one selected from polyoxyalkylene alkyl ether, polyoxyalkylene alkenyl ether, polyoxyethylene castor oil, an alkylene oxide adduct of sorbitan fatty acid ester, and polyoxyethylene hydrogenated castor oil alkanoate.

Examples of the polyoxyalkylene alkyl ether include polyoxyethylene lauryl ether, polyoxyethylene cetyl ether, polyoxyethylene stearyl ether, polyoxyethylene polyoxypropylene lauryl ether, polyoxypropylene cetyl ether, polyoxypropylene isocetyl ether, polyoxypropylene stearyl ether, polyoxyethylene behenyl ether, and the like.

Examples of the polyoxyalkylene alkenyl ether include polyoxyethylene oleyl ether, polyoxypropylene oleyl ether, and the like.

Examples of the polyoxyalkylene alkyl phenyl ether include polyoxyethylene octylphenyl ether, and the like.

The polyoxyethylene castor oil is a compound obtained by addition polymerization of ethylene oxide to a castor oil. An average addition mole number of the ethylene oxide, although not particularly limited, is preferably 2 to 100, and more preferably 10 to 50. Examples of the polyoxyethylene castor oil include NIKKOL CO-3, NIKKOL CO-10, and NIKKOL CO-35 (Nikko Chemicals); EMALEX C-20, EMALEX C-30, EMALEX C-40, and EMALEX C-50 (Nihon Emulsion); Kolliphor EL (BASF); and the like.

Examples of the sorbitan fatty acid ester include sorbitan monooleate, sorbitan monostearate, sorbitan monoisostearate, sorbitan monopalmitate, sorbitan monolaurate, sorbitan trioleate, sorbitan tristearate, sorbitan sesquistearate, sorbitan sesquioleate, sorbitan sesquiisostearate, coconut oil fatty acid sorbitan, and the like. Examples of the alkylene oxide adduct of sorbitan fatty acid ester include polyoxyethylene sorbitan monolaurate, polyoxyethylene sorbitan monopalmitate, polyoxyethylene sorbitan monostearate, polyoxyethylene sorbitan monooleate, polyoxyethylene sorbitan monoisostearate, mono-coconut oil fatty acid polyoxyethylene sorbitan, polyoxyethylene sorbitan tristearate, polyoxyethylene sorbitan trioleate, and the like.

Examples of the polyoxyalkylene fatty acid ester include both polyoxyalkylene monofatty acid ester and polyoxyalkylene difatty acid ester. Examples of the polyoxyalkylene monofatty acid ester include polyethylene glycol monofatty acid esters such as polyethylene glycol monooleate, polyethylene glycol monostearate, and polyethylene glycol monolaurate; and propylene glycol monofatty acid esters such as propylene glycol monostearate, propylene glycol monolaurate, and propylene glycol monooleate.

Examples of the polyoxyethylene hydrogenated castor oil alkanoate include polyoxyethylene hydrogenated castor oil isostearate, polyoxyethylene hydrogenated castor oil stearate, polyoxyethylene hydrogenated castor oil laurate, and the like.

The nanoemulsion in accordance with an embodiment of the present invention only needs to contain at least one of the surfactants, and may contain two or more of the surfactants.

By containing the above surfactant, it is possible to provide a nanoemulsion having stability.

The amount of the surfactant component contained in the nanoemulsion in accordance with an embodiment of the present invention is not particularly limited, provided that it is an amount that allows the nanoemulsion to be formed. The surfactant component is contained in an amount of preferably 10.0% by weight to 49.9% by weight, more preferably 12.0% by weight to 35.0% by weight, and even more preferably 20.0% by weight to 29.0% by weight, relative to the total weight of the nanoemulsion.

The amount of the surfactant component contained in the nanoemulsion is preferably not less than 12.0% by weight, since it makes the average particle diameter sufficiently small. Further, the amount of the surfactant component contained in the nanoemulsion is preferably not more than 29.0% by weight, since it makes the viscosity of the nanoemulsion low.

### (Oil)

The nanoemulsion in accordance with an embodiment of the present invention contains an oil. The oil is not particularly limited, provided that it can be commonly used for edible, pharmaceutical, and cosmetic purposes. Examples of the oil include: naturally derived oils such as avocado oil, olive oil, sesame oil, almond oil, bergamot oil, camellia oil, evening primrose oil, macadamia nut oil, corn oil, rape-seed oil, persic oil, wheat germ oil, camellia sasanqua oil, castor oil, linseed oil, safflower oil, cottonseed oil, perilla oil, soybean oil, peanut oil, tea seed oil, kaya oil, rice bran oil, tung oil, canola oil, red flower oil, kukui nut oil, grape seed oil, hazelnut oil, sunflower oil, rose hip oil, pistachio nut oil, coconut oil, and vegetable squalane; and synthetic oils such as medium chain fatty acid triglyceride, triethylhexanoin, isopropyl myristate, octyldodecyl myristate, hexyldecanol, octyldodecanol, and liquid paraffin. Among those, the naturally derived oils are more preferably vegetable oils. Further, from the viewpoint of absorption into a living body and solubility of functional components such as pharmaceutical drugs, the oil is more preferably soybean oil, medium chain fatty acid triglyceride, squalane, triethylhexanoin, coconut oil, bergamot oil, rose hip oil, and almond oil, and even more preferably squalane and medium chain fatty acid triglyceride. One of the above oils can be used alone. Alternatively, two or more of the above oils can be used in combination. The oil is more preferably an oil having a freezing point of 25°C or less. Vegetable oils containing a large amount of wax components, such as palm oil and jojoba oil, beef tallow, lard, and the like all of which can solidify at room temperature (25°C), are not preferable since they may impair the stability of an emulsion.

The above-described oil is preferably used since it makes it possible to obtain a stable nanoemulsion.

The amount of the oil contained in the nanoemulsion in accordance with an embodiment of the present invention is not particularly limited, provided that it is an amount that allows the nanoemulsion to be formed. The oil is contained in an amount of preferably 0.5% by weight to 5.4% by weight, more preferably 1.5% by weight to 5.0% by weight, and even more preferably 2.0% by weight to 3.5% by weight, relative to the total weight of the nanoemulsion.

The amount of the oil contained in the nanoemulsion is preferably not less than 0.5% by weight, since it makes it possible to stably dissolve a poorly water-soluble functional component. Further, the amount of the oil contained in the nanoemulsion is preferably not more than 5.4% by weight, since it makes the average particle diameter of the nanoemulsion sufficiently small.

### (Poorly water-soluble functional component)

The nanoemulsion in accordance with an embodiment of the present invention contains a poorly water-soluble functional component. In the nanoemulsion in accordance with an embodiment of the present invention, a plurality of types of poorly water-soluble components may be used in combination.

In the present specification, "poorly water-soluble" means "difficult to dissolve", "extremely difficult to dissolve", and "practically insoluble" as defined in the Japanese Pharmacopoeia, and refers to having solubility in water at 20°C of not more than 10 mg/ml. More preferably, "poorly water-soluble" means "extremely difficult to dissolve" and "practically insoluble" as defined in the Japanese Pharmacopoeia, and refers to having solubility in water at 20°C of not more than 1 mg/ml. The poorly water-soluble functional component has a low degree of solubility in water. Thus, when dissolved in the oil to form a nanoemulsion, the poorly water-soluble functional component can be dispersed in an aqueous medium. The poorly water-soluble functional component is preferably fat-soluble and poorly water-soluble.

Further, in the present specification, the "functional component" is not particularly limited, provided that it is a component having some kind of function, and includes, for example, a component that is absorbed in a living body and brings about various functional effects, a component that is contained in a coating agent and brings about various functional effects, and the like. The "functional component" does not necessarily need to be a solid at normal temperature and can also be used in the form of liquid. Examples of a "functional component" in the form of liquid at normal temperature include jojoba oil, squalane, and the like. In a case where the "functional component" also falls under an oil, the nanoemulsion may contain a plurality of oils. In such a case, a highly functional oil corresponds to the "functional component". Alternatively, one type of oil may be contained as a "functional component" and an "oil". In such a case, the "weight ratio of the surfactant component to the oil" and the "weight ratio of the surfactant component to the total weight of the oil and the poorly water-soluble functional component" described later are both intended to mean a "weight ratio of the surfactant component to the one type of oil".

The above-mentioned component that is absorbed in a living body and brings about various functional effects includes, for example, a component that has pharmacological activity for use in inhibiting, diagnosing, alleviating, treating, curing, or preventing a disease or disease condition, a component that provides a predetermined nutritional or health benefit to a living body, and the like. In particular, the poorly water-soluble functional component which is inferior in bioabsorbability as it is has a remarkable effect of providing the composition of the present invention.

The functional component having pharmacological activity is a component used in pharmaceutical products and the like. Examples of the poorly water-soluble functional component having pharmacological activity include, but are not particularly limited to, paclitaxel, cyclosporin, indomethacin, terfenadine, furosemide, acetazolamide, colistin, mebendazole, albendazole, nilotinib, lormetazepam, ibuprofen, mefenamic acid, ketoprofen, bromazepam, and the like.

Examples of the component that provides a nutritional or health benefit include components used in foods for special dietary uses, foods with health claims (foods for specified health uses, foods with nutrient function claims), functional foods, nutrition-supplement foods, health-supplement foods, nutrient-enriched foods, nutrient-adjusted foods, supplements, and the like. Examples of such a poorly water-soluble functional component include coenzyme Q10 (herein also referred to as "CoQ10"), y-oryzanol, curcumin, tocotrienol, testosterone, menthol, carotenoids (α-carotene, β-carotene, lutein, lycopene, astaxanthin, zeaxanthin, cryptoxanthin, fucoxanthin, xanthophyll, and the like), resveratrol, docosahexaenoic acid (DHA), eicosapentaenoic acid (EPA), fat-soluble vitamins (vitamin A, vitamin D, vitamin E (tocopherol), vitamin K), sesamin, α-lipoic acid, saw palmetto extract (oleic acid, lauric acid, myristic acid, linoleic acid, palmitic acid), St. John's wort (hypericin), royal jelly (decenoic acid), hesperidin, nobiletin, quercetin, kaempferol, myricitrin, catechin, daidzein, glycitein, genistein, myricetin, stilbene, and the like, and combinations of two or more of these compounds.

Alternatively, examples of the component that provides a nutritional or health benefit include components used in cosmetic products such as skin improving components, moisturizing components, anti-aging components, hair growth components, and trichogenous components. Examples of such a poorly water-soluble functional component include coenzyme Q10, γ-oryzanol, curcumin, tocotrienol, carotenoids (α-carotene, β-carotene, lutein, lycopene, astaxanthin, zeaxanthin, cryptoxanthin, fucoxanthin, xanthophyll, and the like), resveratrol, docosahexaenoic acid (DHA), eicosapentaenoic acid (EPA), fat-soluble vitamins (vitamin A, vitamin D, vitamin E (tocopherol), vitamin K), ascorbyl tetraisopalmitate, ethylhexyl methoxycinnamate, sesamin, α-lipoic acid, and combinations of two or more of these compounds.

Further, examples of the functional component also include components, such as a color-developing component, used as a coating agent in an industrial field such as printing. Examples of such a poorly water-soluble functional component include coenzyme Q10, curcumin, carotenoids (α-carotene, β-carotene, lutein, lycopene, astaxanthin, zeaxanthin, cryptoxanthin, fucoxanthin, xanthophyll, and the like), and the like, and combinations of two or more of these compounds.

Further, the functional component also includes components used as pesticides such as dichlorodiphenyltrichloroethane (DDT), tebufenpyrad, fenoxycarb, and the like.

The amount of the poorly water-soluble functional component contained in the nanoemulsion in accordance with an embodiment of the present invention is not particularly limited. The poorly water-soluble functional component is contained in an amount of preferably 0.1% by weight to 5.0% by weight, more preferably 0.2% by weight to 4.5% by weight, and even more preferably 0.20% by weight to 3.5% by weight, relative to the total weight of the nanoemulsion.

The amount of the poorly water-soluble functional component contained in the nanoemulsion is preferably not less than 0.1% by weight, since it brings about the effect without lowering the concentration of the poorly water-soluble component. Further, the amount of the poorly water-soluble functional component contained in the nanoemulsion is preferably not more than 4.5% by weight, since it hardly causes precipitation and separation of the poorly water-soluble component.

### (Preservative)

The nanoemulsion in accordance with an embodiment of the present invention contains, as a preservative, at least one selected from an organic acid (salt) and a biguanide-based compound. In the present invention, the "preservative" means an agent used to inhibit propagation of microorganisms and to prevent decay of a nanoemulsion. In the present specification, the "organic acid (salt)" means an organic acid and/or a salt thereof, that is, at least one of an organic acid, a salt thereof, and a mixture thereof.

The organic acid (salt) is not particularly limited, provided that the organic acid (salt) has preservative efficacy. Examples of the organic acid in such organic acids (salts) include sorbic acid, propionic acid, benzoic acid, acetic acid, lactic acid, citric acid, succinic acid, fumaric acid, dehydroacetic acid, and the like. Examples of the salt of the organic acid include sodium salt, potassium salt, aluminum salt, calcium salt, and the like of the organic acids. As the above organic acids (salts), one of the organic acids or salts thereof may be used alone or two or more types of the organic acids and salts thereof may be used in combination.

The organic acid (salt) is more preferably at least one selected from sorbic acid and a salt thereof, and benzoic acid and a salt thereof; even more preferably at least one selected from sodium sorbate, potassium sorbate, sodium benzoate, and potassium benzoate; most preferably at least one selected from potassium sorbate and sodium benzoate.

The above organic acids (salts) have stronger preservative efficacy as the pH decreases. Therefore, a nanoemulsion containing an organic acid (salt) as a preservative is more preferably acidic. The pH of the nanoemulsion is preferably 7.0 or less, more preferably 6.5 or less, and even more preferably 6.0 or less.

In a case where the nanoemulsion in accordance with an embodiment of the present invention contains an organic acid (salt) as a preservative, the nanoemulsion more preferably further contains a pH adjuster for adjusting the pH to the above described range. Examples of such a pH adjuster include, but not particularly limited to, acids such as citric acid and phosphoric acid. The pH adjuster is preferably contained in the form of a pH buffer such as a citric acid-sodium citrate buffer or phosphate buffered saline, from the viewpoint of imparting buffering ability to the nanoemulsion.

In an embodiment of the present invention, the amount of the organic acid (salt) contained is preferably 0.05% by weight to 1.3% by weight, more preferably 0.10% by weight to 1.3% by weight, more preferably 0.20% by weight to 1.0% by weight, even more preferably 0.35% by weight to 0.50% by weight, relative to the total weight of the nanoemulsion. The amount of the organic acid (salt) contained is preferably not less than 0.05% by weight, since such an amount improves the preservative performance of the nanoemulsion. The amount of the organic acid (salt) contained is preferably not more than 1.3% by weight, since such an amount does not cause precipitation of the organic acid (salt) and does not impair the stability of the nanoemulsion.

In an embodiment of the present invention, in a case where the organic acid (salt) is at least one selected from potassium sorbate, sodium dehydroacetate, and sodium benzoate, a contained amount of the potassium sorbate is preferably 0.05% by weight to 0.50% by weight, more preferably 0.10% by weight to 0.50% by weight, even more preferably 0.10% by weight to 0.30% by weight, particularly preferably 0.20% by weight to 0.30% by weight, relative to the total weight of the nanoemulsion; a contained amount of the sodium dehydroacetate is preferably 0.10% by weight to 0.60% by weight, more preferably 0.25% by weight to 0.60% by weight, even more preferably 0.25% by weight to 0.50% by weight, relative to the total weight of the nanoemulsion; and a contained amount of the sodium benzoate is preferably 0.10% by weight to 0.80% by weight, more preferably 0.20% by weight to 0.70% by weight, even more preferably 0.24% by weight to 0.60% by weight, particularly preferably 0.36% by weight to 0.60% by weight, relative to the total weight of the nanoemulsion.

The biguanide-based compound only needs to be any preservative having a biguanide skeleton (N-C(=N)-N-C(=N)-N). Examples of the biguanide-based compound include polyaminopropyl biguanide, chlorhexidine, and the like. As the above biguanide-based compound, one of the biguanide-based compounds may be used alone or two or more types of the biguanide-based compounds may be used in combination.

In an embodiment of the present invention, in a case where the biguanide-based compound is polyaminopropyl biguanide, a contained amount of the polyaminopropyl biguanide is preferably 0.002% by weight to 1.00% by weight, more preferably 0.003% by weight to 0.10% by weight, even more preferably 0.005% by weight to 0.05% by weight, particularly preferably 0.005% by weight to 0.01% by weight, relative to the total weight of the nanoemulsion. The amount of the polyaminopropyl biguanide contained is preferably not less than 0.002% by weight, since it improves the preservative performance of the nanoemulsion. The amount of the polyaminopropyl biguanide contained is preferably not more than 1.00% by weight, since it brings about a small change in color tone of the nanoemulsion.

### (Aqueous medium)

The nanoemulsion in accordance with an embodiment of the present invention contains an aqueous medium. The aqueous medium is not particularly limited, provided that it is a medium containing water. Examples of the aqueous medium include water, bubble water, fruit juice, vegetable juice, soft drinks, milk, yogurt drinks, soy milk, tea drinks, sports drinks, nutrition-supplement drinks, and the like. In the present specification, the "water" can be, for example, so-called water such as ultrapure water (MilliQ (registered trademark) water), distilled water, and ion-exchanged water, and is also intended to include various buffer solutions such as a phosphate buffer solution, physiological saline, and 5% glucose aqueous solution. One of the above aqueous media can be used alone. Alternatively, two or more of the above aqueous media can be used in combination.

The amount of the aqueous medium contained in the nanoemulsion in accordance with an embodiment of the present invention is not particularly limited, provided that it is an amount that allows the nanoemulsion to be formed. The aqueous medium is contained in an amount of preferably 50% by weight to 89.3% by weight, more preferably 60% by weight to 83.5% by weight, and even more preferably 70% by weight to 80.0% by weight, relative to the total weight of the nanoemulsion.

The amount of the aqueous medium contained in the nanoemulsion is preferably not less than 50% by weight, since it makes it possible to suitably form a nanoemulsion. Further, the amount of the aqueous medium contained in the nanoemulsion is preferably not more than 89.3% by weight, since it does not cause an excessively low concentration of the poorly water-soluble functional component.

### (Other components)

The nanoemulsion may further contain, if necessary, an additive other than the above described components, provided that the effect of the present invention is not impaired. Examples of such an additive include an antioxidant, a colorant, a flavoring agent, a thickener, a defoaming agent, and the like.

### (Weight ratio between individual components)

In the nanoemulsion in accordance with an embodiment of the present invention, a weight ratio of the surfactant component to the oil (surfactant component/oil) is preferably 2.80 or more and 240 or less, more preferably 4.50 or more and 240 or less, even more preferably 6.50 or more and 240 or less. A weight ratio of the surfactant component to a total weight of the oil and the poorly water-soluble functional component (surfactant component/(oil+poorly water-soluble functional component)) is preferably 2.60 or more and 200 or less, more preferably 4.00 or more and 200 or less, even more preferably 5.20 or more and 200 or less. The above composition makes it possible to obtain a nanoemulsion having emulsion particles with a small particle diameter, having a narrow particle diameter distribution, and having improved uniformity in particle diameter of the emulsion particles.

In a case where the weight ratio of (surfactant component/oil) is 2.80 or more and the weight ratio of (surfactant component/(oil+poorly water-soluble functional component)) is 2.60 or more, it is possible to obtain a nanoemulsion having emulsion particles with a small particle diameter, having a narrow particle diameter distribution, and having improved uniformity in particle diameter of the emulsion particles. In a case where the weight ratio of (surfactant component/oil) is 6.50 or more and the weight ratio of (surfactant component/(oil+poorly water-soluble functional component)) is 5.20 or more, it is possible to obtain a nanoemulsion having emulsion particles with a smaller particle diameter, having a narrow particle diameter distribution, and having improved uniformity in particle diameter of the emulsion particles. Further, it is preferable that the weight ratio of (surfactant component/oil) is 9.60 or more, and the weight ratio of (surfactant component/(oil+poorly water-soluble functional component)) is 5.20 or more, since it not only brings about the above effect, but also enables the emulsion particles to have a smaller particle diameter.

When LogP of the poorly water-soluble functional component is 4.2 or more, a weight ratio of the oil to the poorly water-soluble functional component (oil/poorly water-soluble functional component) is preferably not less than [-0.38×(LogP of poorly water-soluble functional component)+5.169] and 2000 or less. A lower limit of the weight ratio of the oil to the poorly water-soluble functional component (oil/poorly water-soluble functional component) is more preferably not less than [-0.38×(LogP of poorly water-soluble functional component)+5.9], and even more preferably not less than [-0.38×(LogP of poorly water-soluble functional component)+6.7]. It is preferable that, when the LogP of the poorly water-soluble functional component is 4.2 or more, the lower limit of the weight ratio of the oil to the poorly water-soluble functional component (oil/poorly water-soluble functional component) is not less than [-0.38x(LogP of poorly water-soluble functional component)+5.169], since it provides a sufficient amount of oil for stably dissolving the poorly water-soluble functional component and thus makes it possible to realize a nanoemulsion having excellent stability.

Here, in the present specification, the "LogP" is a common logarithmic value of a 1-octanol-water or 1-octanol-buffer partition coefficient of a chemical substance, and means a value measured by a flask shaking method based on OECD GUIDELINE FOR THE TESTING OF CHEMICALS, "Partition Coefficient (n-octanol/water): Shake Flask Method". A chemical substance having a higher "LogP" value has a higher degree of lipophilicity. In other words, a chemical substance having a higher "LogP" value has a higher degree of fat-solubility.

When the LogP of the poorly water-soluble functional component is less than 4.2, a weight ratio of the oil to the poorly water-soluble functional component (oil/poorly water-soluble functional component) is preferably not less than [-4.955×(LogP of poorly water-soluble functional component)+23.56] and 2000 or less. A lower limit of the weight ratio of the oil to the poorly water-soluble functional component (oil/poorly water-soluble functional component) is more preferably not less than [-4.955×(LogP of poorly water-soluble functional component)+26], and even more preferably not less than [-4.955×(LogP of poorly water-soluble functional component)+29]. It is preferable that, when the LogP of the poorly water-soluble functional component is less than 4.2, the lower limit of the weight ratio of the oil to the poorly water-soluble functional component (oil/poorly water-soluble functional component) is not less than [-4.955×(LogP of poorly water-soluble functional component)+23.56], since it provides a sufficient amount of oil for stably dissolving the poorly water-soluble functional component and thus makes it possible to realize a nanoemulsion having excellent stability.

### (Particle diameter of emulsion particles)

In the nanoemulsion in accordance with an embodiment of the present invention, the surfactant component and the oil form micelles. Specifically, micelles are formed with the poorly water-soluble functional component which is dissolved in the oil and is surrounded by the surfactant component with its hydrophobic group positioned inside. The micelles are emulsion particles of the nanoemulsion.

The emulsion particles have an average particle diameter of preferably 30 nm or less, more preferably 18 nm or less. The emulsion particles preferably have an average particle diameter of 30 nm or less, since it makes it possible to improve the efficiency in absorption of the poorly water-soluble functional component into a living body. Further, a small average particle diameter of the emulsion particles makes it possible to obtain a transparent nanoemulsion. Note here that, in the present specification, the "average particle diameter of the emulsion particles" refers to a value that is measured with use of Zetasizer Nano ZS (available from Malvern Institutes) in the measurement mode "size-small-vol-cell×1.SOP".

Further, the PDI (particle size distribution index) of the emulsion particles is preferably 0.14 or less. The PDI (particle size distribution index) of the emulsion particles is preferably 0.14 or less, since it makes it possible to realize a nanoemulsion that contains emulsion particles which have excellent absorbability into a living body and many of which have an average particle diameter of 30 nm or less and that has excellent stability. Note here that, in the present specification, the "PDI (particle size distribution index) of the emulsion particles" refers to a value that is measured with use of Zetasizer Nano ZS (available from Malvern Institutes) in the "size-small-vol-cell×1.SOP". The polydispersity index (PDI) is also expressed as a particle size distribution index or a polydispersity.

Further, the nanoemulsion is preferably a monodisperse dispersion that does not contain particles of 100 nm or more (subpeaks). The nanoemulsion is preferably a monodisperse dispersion that does not contain particles of 100 nm or more (subpeaks) and that has improved uniformity in particle diameter of the emulsion particles, since it is possible to realize a nanoemulsion that is transparent, has excellent absorbability into a living body, and has excellent stability.

### [2. Method for producing nanoemulsion]

A method for producing the nanoemulsion in accordance with an embodiment of the present invention is not particularly limited. For example, the nanoemulsion in accordance with an embodiment of the present invention can be produced by a production method including: a mixing step of mixing the surfactant component, the oil, and the poorly water-soluble functional component; an aqueous medium addition step of mixing a composition obtained in the mixing step with an aqueous medium; and a preservative addition step of adding a preservative to a composition obtained in the aqueous medium addition step.

Here, the mixing of the surfactant component, the oil, and the poorly water-soluble functional component is preferably carried out such that a weight ratio of the surfactant component to the oil is 2.80 or more and 240 or less, and a weight ratio of the surfactant component to the total weight of the oil and the poorly water-soluble functional component is 2.60 or more and 200 or less.

In the mixing step, the order in which the surfactant component, the oil, and the poorly water-soluble functional component are mixed is not particularly limited. The surfactant component, the oil, and the poorly water-soluble functional component may be all mixed at the same time. Alternatively, the poorly water-soluble functional component may be mixed after the surfactant component and the oil are mixed, the oil may be mixed after the surfactant component and the poorly water-soluble functional component are mixed, or the surfactant component may be mixed after the oil and the poorly water-soluble functional component are mixed.

A method of mixing the individual components described above is not particularly limited, provided that the method can uniformly dissolve the surfactant component, the oil, and the poorly water-soluble functional component. Examples of the method include a method of mixing and stirring the individual components. In the present invention, the individual components can be uniformly dissolved without applying a particularly strong shearing force. For example, the individual components can be uniformly dissolved by stirring them with a magnetic stirrer.

Further, a temperature at which the individual components are mixed is not particularly limited, but a liquid temperature is more preferably 20°C or more and 90°C or less, and even more preferably 30°C or more and 75°C or less.

The step of mixing the composition obtained in the mixing step and the aqueous medium is a step of adding the aqueous medium to the composition obtained in the mixing step. This step may involve adding the aqueous medium in its entirety at a time or in fractional amounts while the mixture is being stirred.

A method of mixing the composition obtained in the mixing step and the aqueous medium is not particularly limited, provided that the method can disperse the composition as emulsion particles in the aqueous medium. Examples of the method include a method of mixing and stirring the composition and the aqueous medium. In this step as well, the composition can be dispersed as emulsion particles in the aqueous medium without applying a particularly strong shearing force. For example, the composition and the aqueous medium can be stirred with a magnetic stirrer.

Further, the temperature at which the composition and the aqueous medium are mixed is not particularly limited, but a liquid temperature is more preferably 20°C or more and 90°C or less, and even more preferably 30°C or more and 75°C or less.

The preservative addition step is a step of adding a preservative to the composition obtained in the aqueous medium addition step. This step may involve adding the aqueous medium in its entirety at a time or in fractional amounts while the mixture is being stirred.

A method of adding a preservative to the composition obtained in the aqueous medium addition step is not particularly limited. Examples of the method include a method of mixing and stirring the composition and the preservative. For example, the composition and the preservative can be stirred with a magnetic stirrer.

Further, the temperature at which the composition and the preservative are mixed is not particularly limited, but a liquid temperature is preferably 5°C or more and 55°C or less, and more preferably 10°C or more and 50°C or less.

The preservative addition step does not necessarily need to be carried out after the aqueous medium addition step. The preservative may be added in the mixing step of mixing the oil with the poorly water-soluble functional component, or in the aqueous medium addition step of mixing the composition obtained in the mixing step with an aqueous medium.

### [4. Use of nanoemulsion]

The present invention also includes a pharmaceutical product, a functional food, a cosmetic product, a coating agent, or a pesticide, each containing the nanoemulsion in accordance with an embodiment of the present invention.

The present invention is not limited to the embodiments, but can be altered by a skilled person in the art within the scope of the claims. The present invention also encompasses, in its technical scope, any embodiment derived by combining technical means disclosed in differing embodiments.

An embodiment of the present invention encompasses the following features.
<1> A nanoemulsion which contains emulsion particles that have an average particle diameter of 30 nm or less and have a particle size distribution index of 0.14 or less, and which does not contain emulsion particles of 100 nm or more, the nanoemulsion containing a surfactant component, an oil, a poorly water-soluble functional component, an aqueous medium, and a preservative, the preservative containing at least one selected from an organic acid (salt) and a biguanide-based compound.
<2> The nanoemulsion described in <1>, in which: the organic acid (salt) contains at least one selected from sorbic acid and a salt thereof, benzoic acid and a salt thereof, and dehydroacetic acid and a salt thereof; and the nanoemulsion further contains a pH adjuster.
<3> The nanoemulsion described in <1> or <2>, in which: the organic acid (salt) contains at least one selected from potassium sorbate, sodium benzoate, and sodium dehydroacetate; a contained amount of the potassium sorbate is 0.05% by weight to 0.50% by weight, relative to a total weight of the nanoemulsion; a contained amount of the sodium benzoate is 0.10% by weight to 0.80% by weight, relative to the total weight of the nanoemulsion; a contained amount of the sodium dehydroacetate is 0.10% by weight to 0.60% by weight, relative to the total weight of the nanoemulsion; and a pH of the nanoemulsion is 6.0 or less.
<4> The nanoemulsion described in any one of <1> through <3>, in which: the organic acid (salt) contains sodium benzoate in an amount of 0.36% by weight to 0.60% by weight, relative to a total weight of the nanoemulsion.
<5> The nanoemulsion described in any one of <1> through <4>, in which: the biguanide-based compound contains polyaminopropyl biguanide.
<6> The nanoemulsion described in any one of <1> through <5>, in which: the biguanide-based compound contains polyaminopropyl biguanide in an amount of 0.002% by weight to 1.00% by weight, relative to a total weight of the nanoemulsion.
<7> The nanoemulsion described in any one of <1> through <6>, in which: a weight ratio of the surfactant component to the oil is 2.80 or more and 240 or less; and a weight ratio of the surfactant component to a total weight of the oil and the poorly water-soluble functional component is 2.60 or more and 200 or less.
<8> The nanoemulsion described in any one of <1> through <7>, in which: the surfactant component contains at least one selected from polyoxyalkylene alkyl ether, polyoxyalkylene alkenyl ether, a polyoxyethylene castor oil, an alkylene oxide adduct of sorbitan fatty acid ester, and polyoxyethylene hydrogenated castor oil alkanoate.
<9> A pharmaceutical product, a functional food, a cosmetic product, a coating agent, or a pesticide, each containing a nanoemulsion described in any one of <1> through <8>.

### Examples

The following will describe the present invention in greater detail on the basis of Examples. The present invention is, however, not limited to the Examples below.

### [Evaluation method]

Nanoemulsions obtained in Examples and Comparative Examples were evaluated by the following methods.

(Measurements of average particle diameter, PDI, and particle diameter distribution (presence or absence of subpeak) of emulsion particles in nanoemulsion) The nanoemulsions obtained in Examples and Comparative Examples were each diluted 50-fold to 100-fold with ion-exchanged water to prepare samples for measurement. The PDI was measured with use of Zetasizer Nano ZS (available from Malvern Panalytical Ltd.). Further, the particle diameter of the emulsion particles was measured on the basis of a Z-average value.

The presence or absence of subpeaks was determined from a particle diameter distribution graph obtained as the results of the measurement. If there are any subpeaks, the measurement results are automatically outputted as Peak-2, Peak-3, etc.

### (Preservative property of nanoemulsion)

In a method A, 20 g of a nanoemulsion was taken in a sterilized vial, and was inoculated with 0.2 mL of a test bacterial liquid (kitchen waste liquid). The sample inoculated with the test bacterial liquid was kept at 30°C, and the number of living bacteria on the 21st day was measured. The evaluation criterion was as follows.

Excellent: The number of living bacteria on the 21st day is less than 0.01% of the number of living bacteria after inoculation.

Good: The number of living bacteria on the 21st day is 0.01% or more and less than 100% of the number of living bacteria after inoculation.

Poor: The number of living bacteria on the 21st day is 100% or more of the number of living bacteria after inoculation.

In a method B, 20 g of a nanoemulsion was taken in a sterilized vial, and was inoculated with 0.15 mL of each of five types of test bacterial liquids, i.e., *Escherichia coli, Pseudomonas aeruginosa, Staphylococcus aureus, Candida,* and *Aspergillus niger.* The five samples thus obtained were kept at 22.5°C, and the number of living bacteria on the 28th day was measured. The evaluation criterion was as follows.

Excellent: The number of living bacteria on the 28th day is less than 0.01% of the number of living bacteria after inoculation for all the five types of test bacteria.

Good: The number of living bacteria on the 28th day is 0.01% or more and less than 100% of the number of living bacteria after inoculation for at least one of the five types of test bacteria and, for none of the five types of test bacteria, the number of living bacteria on the 28th day is 100% or more of the number of living bacteria after inoculation.

Poor: The number of living bacteria on the 28th day is 100% or more of the number of living bacteria after inoculation for at least one of the five types of test bacteria.

### (Stability of nanoemulsion)

The nanoemulsions obtained in Examples and Comparative Examples were each sealed in a colorless and transparent glass bottle and left to stand under conditions of 4°C, room temperature, and 40°C, and visually observed after 21 days from the start of leaving to stand. The evaluation criterion was as follows.

Good: The nanoemulsion is uniform and transparent under all conditions of 4°C, room temperature, and 40°C.

Poor: Two-layer separation of a liquid layer, floatation of a precipitate, precipitation, or a subpeak is observed under at least one of the conditions of 4°C, room temperature, and 40°C.

### [Production of nanoemulsion]

### [Components]

The following materials were used as components constituting a nanoemulsion.

### (Surfactant)

- Polyoxyethylene (9) oleyl ether (available from Aoki Oil Industrial Co., Ltd., Blaunon EN-909)
- Polyoxyethylene (7) lauryl ether (available from Aoki Oil Industrial Co., Ltd., Blaunon EL-1507)
- Polyoxyethylene (35) castor oil (available from Nikko Chemicals Co., Ltd., NIKKOL CO-35)
- Polysorbate 80 (available from Kao Corporation, RHEODOL TW-O120V)
- PEG-50 hydrogenated castor oil isostearate (available from Nihon Emulsion Co., Ltd., EMALEX RWIS-150EX)

### (Oil)

- Squalane (available from Oryza Oil & Fat Chemical Co., Ltd., ORYZA SQUALANE)
- Medium chain fatty acid triglyceride (available from Kao Corporation, COCONARD MT)
- Triethylhexanoin (available from The Nisshin OilliO Group, Ltd., T.I.O)
- Rice bran oil (available from Oryza Oil & Fat Chemical Co., Ltd., Rice bran oil)

### (Poorly water-soluble functional component)

- γ-oryzanol (available from Oryza Oil & Fat Chemical Co., Ltd., γ-oryzanol)
- Coenzyme Q10 (available from FUJIFILM Wako Pure Chemical Corporation, Ubiquinone 10)
- Stearyl glycyrrhetinate (available from MARUZEN PHARMACEUTICALS CO., LTD., CO Gretinol (registered trademark))
- Resveratrol (available from Tokyo Chemical Industry Co., Ltd., Resveratrol)

### (Preservative)

- Sodium benzoate (available from Tokyo Chemical Industry Co., Ltd., Sodium Benzoate)
- Potassium sorbate (available from Tokyo Chemical Industry Co., Ltd., Potassium Sorbate)
- Sodium dehydroacetate (available from Tokyo Chemical Industry Co., Ltd., Sodium Dehydroacetate Monohydrate)
- Polyaminopropyl piguanide (available from Nikko Chemicals Co., Ltd., COSMOCIL CQ)
- Methylparaben (available from Tokyo Chemical Industry Co., Ltd., Methyl 4-Hydroxybenzoate)
- Butylparaben (available from Tokyo Chemical Industry Co., Ltd., Butyl 4-Hydroxybenzoate)
- Phenoxyethanol (available from Nacalai Tesque, Inc., Ethylene glycol monophenyl ether)
- 1,2-propanediol (available from Nacalai Tesque, Inc., propylene glycol)
- 1,3-butanediol (available from Nacalai Tesque, Inc., 1,3-butanediol)
- Ethylhexylglycerin (SEIWA SUPPLY CO., LTD., sensiva SC 50 JP)

### (pH adjuster)

- Citric acid-sodium citrate buffer (pH=4.8)
   This buffer was prepared as follows: citric acid monohydrate and trisodium citrate dihydrate (both available from FUJIFILM Wako Pure Chemical Corporation) were mixed in ion-exchanged water such that the pH was 4.8 and the sum of concentrations of citric acid and sodium citrate was 0.1 mol/L.
- Citric acid-sodium citrate buffer (pH=5.6)
   This buffer was prepared as follows: citric acid monohydrate and trisodium citrate dihydrate were mixed in ion-exchanged water such that the pH was 5.6 and the sum of concentrations of citric acid and sodium citrate was 0.1 mol/L.

### [Example 1]

In a beaker, polyoxyethylene (9) oleyl ether and polyoxyethylene (7) lauryl ether as surfactants, squalane as an oil, and γ-oryzanol as a poorly water-soluble component were weighed at the weight ratios shown in Table 1. The weighed mixture in the beaker was stirred at 70°C with a magnetic stirrer until the whole of the poorly water-soluble functional component was uniformly dissolved by a magnetic stirring bar, so that a nanoemulsion preparation composition, which was a viscous liquid, was obtained. To the nanoemulsion preparation composition, a citric acid-sodium citrate buffer (pH=4.8) as a pH adjuster and an aqueous medium in the nanoemulsion was added so as to have the weight ratio shown in Table 1, and the mixture was stirred at 70°C for another 1 to 3 minutes. After it was visually confirmed that there was no uneven undissolved residue or lump, the mixture was cooled to room temperature in an ice water tank to obtain a nanoemulsion. Further, a preservative aqueous solution prepared by mixing sodium benzoate and ion-exchanged water at the weight ratios shown in Table 1 was added to the nanoemulsion to obtain a preservative-added nanoemulsion. The preservative property of the preservative-added nanoemulsion, as well as the average particle diameter and PDI of the emulsion particles in the nanoemulsion were evaluated. The results are shown in Table 1.

**[Table 1]**

| Classification | Component name | | | Example 1 | Example 2 | Example 3 | Example 4 | Example 5 | Example 6 | Example 7 | Example 8 | Example 9 |
|---|---|---|---|---|---|---|---|---|---|---|---|---|
| pH adjuster | Citric acid-sodium citrate buffer (pH=4.8) | | | 73.80 | 72.60 | 70.21 | | | 75.22 | 74.20 | 73.20 | |
| | Citric acid-sodium citrate buffer (pH=5.6) | | | | | | 73.66 | 70.20 | | | | 75.10 |
| Surfactant | Polyoxyethylene (9) oleyl ether | | | 10.00 | 10.00 | 10.00 | 10.01 | 10.00 | 9.99 | 10.00 | 10.00 | 10.00 |
| | Polyoxyethylene (7) lauryl ether | | | 10.00 | 10.00 | 10.00 | 10.01 | 10.00 | 9.99 | 10.00 | 10.00 | 10.00 |
| Oil | Squalane | | | 3.00 | 3.00 | 3.00 | 3.00 | 3.00 | 3.00 | 3.00 | 3.00 | 3.00 |
| Poorly water-soluble functional component | *γ* -oryzanol | | | 0.80 | 0.80 | 0.80 | 0.80 | 0.80 | 0.80 | 0.80 | 0.80 | 0.80 |
| Preservative aqueous solution | Sodium benzoate | | | 0.24 | 0.36 | 0.59 | 0.36 | 0.60 | | | | |
| | Potassium sorbate | | | | | | | | 0.10 | 0.20 | 0.30 | 0.20 |
| | Sodium dehydroacetate | | | | | | | | | | | |
| | Ion-exchanged water | | | 2.16 | 3.24 | 5.40 | 2.16 | 5.40 | 0.90 | 1.80 | 2.70 | 0.90 |
| | pH | | | 5.1 | 5.1 | 5.5 | 5.8 | 5.9 | 4.9 | 5.1 | 5.4 | 5.9 |
| | Particle diameter (nm) | | | 10.90 | 11.18 | 10.90 | 11.11 | 11.01 | 11.93 | 11.44 | 10.93 | 11.23 |
| | Particle size distribution index PDI | | | 0.059 | 0.055 | 0.024 | 0.002 | 0.063 | 0.099 | 0.090 | 0.055 | 0.059 |
| | Subpeaks with average particle diameter of 100 nm or more (%) | | | 0 | 0 | 0 | 0 | 0 | 0 | 0 | 0 | 0 |
| | Surfactant/(oil+poorly water-soluble component) | | | 5.26 | 5.26 | 5.26 | 5.27 | 5.26 | 5.26 | 5.26 | 5.26 | 5.26 |
| | LogP of poorly water-soluble component | | | 12 | 12 | 12 | 12 | 12 | 12 | 12 | 12 | 12 |
| | -0.38 × [LogP]+5.169 | | | 0.609 | 0.609 | 0.609 | 0.609 | 0.609 | 0.609 | 0.609 | 0.609 | 0.989 |
| | Preservative property test | Method A | After inoculation (pieces/g) | 140,000 | 140,000 | 360,000 | 360,000 | 360,000 | 140,000 | 140,000 | 360,000 | 360,000 |
| | | | After 21 days (pieces/g) | 8,000 | <10 | <10 | 172,000 | 64,000 | 3,300 | <10 | <10 | 67,000 |
| | | | Preservative property result | Good | Excellent | Excellent | Good | Good | Good | Excellent | Excellent | Good |
| | | Method B | Preservative property result | Good | Good | Good | Good | Good | Good | Good | Good | Good |
| | Stability after 21 days | | | Good | Good | Good | Good | Good | Good | Good | Good | Good |

**Continued from Table 1**

| Classification | Component name | | | Example 10 | Example 11 | Example 12 | Example 13 | Example 14 | Example 15 | Comparative Example 1 |
|---|---|---|---|---|---|---|---|---|---|---|
| pH adjuster | Citric acid-sodium citrate buffer (pH=4.8) | | | | 72.80 | 73.70 | 71.20 | | | 76.19 |
| | Citric acid-sodium citrate buffer (pH=5.6) | | | 73.20 | | | | 73.70 | 71.20 | |
| Surfactant | Polyoxyethylene (9) oleyl ether | | | 10.00 | 10.00 | 10.00 | 10.00 | 10.00 | 10.00 | 10.00 |
| | Polyoxyethylene (7) lauryl ether | | | 10.00 | 10.00 | 10.00 | 10.00 | 10.00 | 10.00 | 10.00 |
| Oil | Squalane | | | 3.00 | 3.00 | 3.00 | 3.00 | 3.00 | 3.00 | 3.01 |
| Poorly water-soluble functional component | γ-oryzanol | | | 0.80 | 0.80 | 0.80 | 0.80 | 0.80 | 0.80 | 0.80 |
| Preservative aqueous solution | Sodium benzoate | | | | 0.24 | | | | | |
| | Potassium sorbate | | | 0.30 | 0.10 | | | | | |
| | Sodium dehydroacetate | | | | | 0.25 | 0.50 | 0.25 | 0.50 | |
| | Ion-exchanged water | | | 2.70 | 3.06 | 2.25 | 4.50 | 2.25 | 4.50 | |
| | pH | | | 5.9 | 5.2 | 5.3 | 5.3 | 5.8 | 5.9 | 5.4 |
| | Particle diameter (nm) | | | 11.07 | 11.56 | 10.63 | 10.74 | 11.88 | 11.80 | 11.44 |
| | Particle size distribution index PDI | | | 0.033 | 0.033 | 0.055 | 0.086 | 0.035 | 0.047 | 0.015 |
| | Subpeaks with average particle diameter of 100 nm or more (%) | | | 0 | 0 | 0 | 0 | 0 | 0 | 0 |
| | Surfactant/(oil+poorly water-soluble component) | | | 5.26 | 5.26 | 5.26 | 5.26 | 5.26 | 5.26 | 5.25 |
| | LogP of poorly water-soluble component | | | 12 | 12 | 12 | 12 | 12 | 12 | 12 |
| | -0.38 × [LogP]+5.169 | | | 0.609 | 0.609 | 0.609 | 0.609 | 0.609 | 0.609 | 0.609 |
| | Preservative property test | Method A | After inoculation (pieces/g) | 360,000 | 140,000 | 360,000 | 360,000 | 360,000 | 360,000 | 260,000 |
| | | | After 21 days | 23,000 | <10 | <10 | <10 | <10 | <10 | 2,600,000 |
| | | | Preservative property result | Good | Excellent | Excellent | Excellent | Excellent | Excellent | Poor |
| | | Method B | Preservative property result | Good | Good | Good | Excellent | Good | Good | Poor |
| | Stability after 21 days | | | Good | Good | Good | Good | Good | Good | Good |

### [Examples 2 through 5]

Nanoemulsions were each prepared in a manner similar to that in Example 1, except that a citric acid-sodium citrate buffer (pH=4.8 or pH=5.6) was used as a pH adjuster and the weight ratios of the components were changed to the weight ratios shown in Table 1. The obtained nanoemulsions were evaluated. The results are shown in Table 1.

### [Examples 6 through 10]

Nanoemulsions were each prepared in a manner similar to that in Example 1, except that potassium sorbate was used in place of sodium benzoate as a preservative, a citric acid-sodium citrate buffer (pH=4.8 or pH=5.6) was used as a pH adjuster, and the weight ratios of the components were changed to the weight ratios shown in Table 1. The obtained nanoemulsions were evaluated. The results are shown in Table 1.

### [Example 11]

A nanoemulsion was prepared in a manner similar to that in Example 1, except that sodium benzoate and potassium sorbate were used as preservatives, and the weight ratios of the components were changed to the weight ratios shown in Table 1. The obtained nanoemulsion was evaluated. The results are shown in Table 1.

### [Examples 12 through 15]

Nanoemulsions were each prepared in a manner similar to that in Example 1, except that sodium dehydroacetate was used in place of sodium benzoate as a preservative, a citric acid-sodium citrate buffer (pH=4.8 or pH=5.6) was used as a pH adjuster, and the weight ratios of the components were changed to the weight ratios shown in Table 1. The obtained nanoemulsions were evaluated. The results are shown in Table 1.

### [Comparative Example 1]

A nanoemulsion was prepared in a manner similar to that in Example 1, except that no preservative was added and the weight ratios of the components were changed to the weight ratios shown in Table 1. The obtained nanoemulsion was evaluated. The results are shown in Table 1.

### [Result 1]

As shown in Table 1, in a case where the organic acid (salt) was used as a preservative, the number of living bacteria on the 21st day in the method A was less than 100% of the number of living bacteria after inoculation. The number of living bacteria on the 28th day was 0.01% or more and less than 100% of the number of living bacteria after inoculation for at least one of the five types of test bacteria in the method B and, for none of the five types of test bacteria, the number of living bacteria on the 28th day was 100% or more of the number of living bacteria after inoculation.

In particular, in a case where sodium benzoate was used and a weight ratio thereof was not less than 0.36% by weight and not more than 0.59% by weight, or potassium sorbate was used and a weight ratio thereof was not less than 0.20% by weight and not more than 0.30% by weight, and the pH was 5.5 or less, or in a case where sodium dehydroacetate was used and a weight ratio thereof was not less than 0.25% by weight and not more than 0.50% by weight, and the pH was 6.0 or less, the number of living bacteria on the 21st day in the method A was less than 0.01% of the number of living bacteria after inoculation.

In a case where sodium benzoate and potassium sorbate were used in combination, the number of living bacteria on the 21st day in the method A was less than 0.01% of the number of living bacteria after inoculation, the number of living bacteria on the 28th day was 0.01% or more and less than 100% of the number of living bacteria after inoculation for at least one of the five types of test bacteria in the method B and, for none of the five types of test bacteria, the number of living bacteria on the 28th day was 100% or more of the number of living bacteria after inoculation.

Further, in a visual observation of the nanoemulsion on the 21st day, the liquid state was uniform and transparent.

That is, it can be seen that, in a case of containing, as an organic acid (salt), at least one selected from the sodium benzoate, the potassium sorbate, and the sodium dehydroacetate, it is possible to obtain a stable nanoemulsion having preservative efficacy, and one of the organic acids (salts) can be used, or two or more of the organic acids (salts) can be used.

### [Example 16]

A nanoemulsion was prepared in a manner similar to that in Example 1, except that polysorbate 80 was used in place of polyoxyethylene (9) oleyl ether and polyoxyethylene (7) lauryl ether as a surfactant, medium chain fatty acid triglyceride was used in place of squalane as an oil, coenzyme Q10 was used in place of γ-oryzanol as a poorly water-soluble functional component, and the weight ratios of the components were changed to the weight ratios shown in Table 2. The obtained nanoemulsion was evaluated. The results are shown in Table 2.

**[Table 2]**

| Classification | Component name | | | Example 16 | Comparative Example 2 |
|---|---|---|---|---|---|
| pH adjuster | Citric acid-sodium citrate buffer (pH=4.8) | | | 68.69 | 72.29 |
| Surfactant | Polysorbate 80 | | | 25.01 | 25.01 |
| Oil | Medium chain fatty acid triglyceride | | | 2.50 | 2.50 |
| Poorly water-soluble functional component | Coenzyme Q10 | | | 0.20 | 0.20 |
| Preservative aqueous solution | Sodium benzoate | | | 0.36 | |
| | Ion-exchanged water | | | 3.24 | |
| | pH | | | 5.3 | 5.3 |
| | Particle diameter (nm) | | | 11.05 | 10.67 |
| | Particle size distribution index PDI | | | 0.016 | 0.059 |
| | Subpeaks with average particle diameter of 100 nm or more (%) | | | 0 | 0 |
| | Surfactant/(oil+poorly water-soluble component) | | | 9.26 | 9.26 |
| | LogP of poorly water-soluble component | | | 11 | 11 |
| | -0.38 × [LogP]+5.169 | | | 0.989 | 0.989 |
| | Preservative property test | Method A | After inoculation (pieces/g) | 260,000 | 260,000 |
| | | | After 21 days (pieces/g) | 100 | 2,400,000 |
| | | | Preservative property result | Good | Poor |
| | | Method B | Preservative property result | Good | Poor |
| | Stability after 21 days | | | Good | Good |

### [Comparative Example 2]

A nanoemulsion was prepared in a manner similar to that in Example 16, except that no preservative was added and the weight ratios of the components were changed to the weight ratios shown in Table 2. The obtained nanoemulsion was evaluated. The results are shown in Table 2.

### [Result 2]

As shown in Table 2, in a case where the organic acid (salt) was used as a preservative, even when the types of surfactant and oil were changed, the number of living bacteria on the 21st day in the method A was 0.01% or more and less than 100% of the number of living bacteria after inoculation, the number of living bacteria on the 28th day was 0.01% or more and less than 100% of the number of living bacteria after inoculation for at least one of the five types of test bacteria in the method B and, for none of the five types of test bacteria, the number of living bacteria on the 28th day was 100% or more of the number of living bacteria after inoculation. It can be seen that, even when the types of surfactant and oil are changed, a stable nanoemulsion having preservative efficacy can be obtained, provided that the nanoemulsion contains the above described preservative. Further, it can be seen that the nanoemulsion that does not contain the above described preservative has no preservative efficacy, as compared with the nanoemulsion that contains the preservative.

### [Examples 17 and 18]

Nanoemulsions were each prepared in a manner similar to that in Example 1, except that polyaminopropyl piguanide was used in place of sodium benzoate as a preservative, ion-exchanged water was used in place of the citric acid-sodium citrate buffer (pH=4.8), and the weight ratios of the components were changed to the weight ratios shown in Table 3. The obtained nanoemulsions were evaluated. The results are shown in Table 3.

**[Table 3]**

| Classification | Component name | | | Example 17 | Example 18 | Example 19 | Example 20 | Comparative Example 3 |
|---|---|---|---|---|---|---|---|---|
| | Ion-exchanged water (1) | | | 76.10 | 75.90 | 71.94 | 71.90 | 72.00 |
| Surfactant | Polyoxyethylene (9) oleyl ether | | | 10.00 | 10.00 | | | |
| | Polyoxyethylene (7) lauryl ether | | | 10.00 | 10.00 | | | |
| | Polyoxyethylene (35) castor oil | | | | | 25.00 | 24.99 | 25.00 |
| Oil | Squalane | | | 3.00 | 3.00 | | | |
| | Medium chain fatty acid triglyceride | | | | | 2.51 | 2.51 | 2.50 |
| Poorly water-soluble functional component | *γ* -oryzanol | | | 0.80 | 0.80 | | | |
| | Coenzyme Q10 | | | | | 0.50 | 0.50 | 0.50 |
| Preservative aqueous solution | Polyaminopropyl biguanide | | | 0.010 | 0.030 | 0.005 | 0.010 | |
| | Ion-exchanged water (2) | | | 0.09 | 0.27 | 0.045 | 0.09 | |
| | pH | | | 6.5 | 6.5 | 6.5 | 6.5 | 6.6 |
| | Particle diameter (nm) | | | 10.94 | 11.01 | 16.44 | 16.56 | 15.55 |
| | Particle size distribution index PDI | | | 0.039 | 0.075 | 0.029 | 0.06 | 0.014 |
| | Subpeaks with average particle diameter of 100 nm or more (%) | | | 0 | 0 | 0 | 0 | 0 |
| | Surfactant/(oil+poorly water-soluble component) | | | 5.26 | 5.26 | 8.31 | 8.30 | 8.33 |
| | LogP of poorly water-soluble component | | | 12 | 12 | 11 | 11 | 11 |
| | -0.38 × [LogP]+5.169 | | | 0.609 | 0.609 | 0.989 | 0.989 | 0.989 |
| | Preservative property test | Method A | After inoculation (pieces/g) | 140,000 | 360,000 | 460,000 | 460,000 | 260,000 |
| | | | After 21 days (pieces/g) | <10 | <10 | <10 | <10 | 2,800,000 |
| | | | Preservative property result | Excellent | Excellent | Excellent | Excellent | Poor |
| | | Method B | Preservative property result | Good | Good | Good | Good | Poor |
| | Stability after 21 days | | | Good | Good | Good | Good | Good |

### [Examples 19 and 20]

Nanoemulsions were each prepared in a manner similar to that in Example 17, except that polyaminopropyl piguanide was used as a preservative, a polyoxyethylene (35) castor oil was used in place of polyoxyethylene (9) oleyl ether and polyoxyethylene (7) lauryl ether as a surfactant, coenzyme Q10 was used in place of γ-oryzanol as a poorly water-soluble functional component, and the weight ratios of the components were changed to the weight ratios shown in Table 3. The obtained nanoemulsions were evaluated. The results are shown in Table 3.

### [Comparative Example 3]

A nanoemulsion was prepared in a manner similar to that in Example 19, except that no preservative was added and the weight ratios of the components were changed to the weight ratios shown in Table 3. The obtained nanoemulsion was evaluated. The results are shown in Table 3.

### [Result 3]

As shown in Table 3, in a case where the biguanide-based compound was used as a preservative, regardless of the surfactant, the oil, and the poorly water-soluble functional component, the number of living bacteria on the 21st day in the method A was less than 0.01% of the number of living bacteria after inoculation, the number of living bacteria on the 28th day was 0.01% or more and less than 100% of the number of living bacteria after inoculation for at least one of the five types of test bacteria in the method B and, for none of the five types of test bacteria, the number of living bacteria on the 28th day was 100% or more of the number of living bacteria after inoculation.

Further, in an observation of the nanoemulsion on the 21st day, the liquid state was uniform and transparent.

Further, it can be seen that, in a case where polyaminopropyl biguanide is contained as the biguanide-based compound and the amount of the polyaminopropyl biguanide contained is preferably 0.005% by weight to 0.03% by weight, relative to the total weight of the nanoemulsion, it is possible to obtain a stable nanoemulsion having preservative efficacy.

### [Comparative Examples 4 through 14]

Nanoemulsions were each prepared in a manner similar to that in Example 1, except that ion-exchanged water was used in place of the citric acid-sodium citrate buffer (pH=4.8), and the components and weight ratios of the surfactant, the oil, the poorly water-soluble functional component, and the preservative were changed to those shown in Table 4. The obtained nanoemulsions were evaluated. The results are shown in Table 4.

**[Table 4]**

| Classification | Component name | | | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 | Comparative Examp e 14 |
|---|---|---|---|---|---|---|---|---|---|---|---|---|---|---|
| | Ion-exchanged water (1) | | | 66.27 | 63.32 | 66.29 | 63.41 | 64.50 | 66.00 | 64.50 | 66.00 | 63.80 | 65.49 | 64.99 |
| Surfactant | Polyoxyethylene (35) castor oil | | | 24.97 | 24.95 | | | | | | | | | |
| | Polysorbate 80 | | | | | 24.99 | 24.99 | 28.00 | 28.00 | 28.00 | 28.00 | 28.00 | | |
| | Polyoxyethylene (9) oleyl ether | | | | | | | | | | | | 10.01 | 10.C0 |
| | Polyoxyethylene (7) lauryl ether | | | | | | | | | | | | 10.01 | 10.00 |
| Oil | Medium chain fatty acid triglyceride | | | 2.54 | 2.53 | 2.51 | 2.51 | 3.00 | 3.00 | 3.00 | 3.00 | 3.00 | 2.99 | |
| | Squalane | | | | | | | | | | | | | 3.01 |
| Poorly water-soluble functional component | Coenzyme Q10 | | | 0.50 | 0.50 | 0.50 | 0.50 | | | | | | | |
| | Stearyl glycyrrhetinate | | | | | | | | | | | | 0.30 | |
| | *γ* -oryzanol | | | | | | | | | | | | | 0.80 |
| Preservative or Preservative aqueous solution | Methylparaben | | | 0.20 | 0.21 | 0.20 | 0.20 | 0.15 | | | 0.20 | 0.13 | | |
| | Butylparaben | | | | 0.10 | | 0.15 | 0.15 | 0.15 | 0.23 | | | | |
| | Phenoxyethanol | | | 0.10 | 0.15 | 0.10 | 0.10 | 0.15 | 0.15 | 0.22 | 0.10 | 0.39 | 1.20 | 1.20 |
| | 1,2-propanediol | | | 2.71 | 4.12 | 2.70 | 4.07 | | | | | | | |
| | 1,3-butanediol | | | | | | | | | | | | 10.00 | 10.00 |
| | Ethylhexylglycerin | | | | | | | 4.06 | 2.70 | 4.06 | 2.71 | 4.68 | | |
| | Ion-exchanged water (2) | | | 2.71 | 4.12 | 2.70 | 4.07 | | | | | | | |
| | pH | | | 7.1 | 7.0 | 7.0 | 7.0 | 6.9 | 7.0 | 7.0 | 6.9 | 6.9 | 6.9 | 7.0 |
| | Particle diameter (nm) | | | 15.97 | 15.67 | 11.01 | 11.20 | 9.333 | 9.510 | 10.01 | 10.20 | 9.293 | 9.293 | 11.43 |
| | Particle size distribution index PD! | | | 0.039 | 0.016 | 0.056 | 0.03 | 0.038 | 0.068 | 0.042 | 0.037 | 0.072 | 0.072 | 0.025 |
| | Subpeaks with average particle diameter of 100 nm or more (%) | | | 0 | 0 | 0 | 0 | 0 | 0 | 0 | 0 | 0 | 0 | 0 |
| | Surfactant/(oil+poorly water-soluble component) | | | 8.23 | 8.23 | 8.30 | 8.30 | 9.34 | 9.34 | 9.34 | 9.34 | 9.34 | 0.08 | 6.64 |
| | LogP of poorly water-soluble component | | | 11 | 11 | 11 | 11 | - | - | - | - | - | 16 | 12 |
| | -0.38× [LogP]+5.169 | | | 0.989 | 0.989 | 0.989 | 0.989 | - | - | - | - | - | -0.911 | 0.609 |
| | Preservative property test | Method A | After inoculation (pieces/g) | 220,000 | 220,000 | 220,000 | 220,000 | 200,000 | 200,000 | 200,000 | 200,000 | 200,000 | 460,000 | 260,000 |
| | | | After 21 days (pieces/g) | <10 | 40,000 | 520,000 | <10 | 510,000 | 990,000 | 620,000 | 830,000 | 1,000,000 | <10 | <10 |
| | | | Preservative property result | Excellent | Good | Poor | Excellent | Poor | Poor | Poor | Poor | Poor | Excellent | Excellent |
| | | Method B | Preservative property result | Good | Good | Poor | Good | Poor | Poor | Poor | Poor | Poor | Good | Good |
| | Stability after 21 days | | | Poor | Poor | Poor | Poor | Good | Good | Good | Good | Good | Poor | Poor |

### [Result 4]

As shown in Table 4, in Comparative Examples 4, 5, 7, 13, and 14, preservative property was observed but no stability was observed. This seems to be because it is necessary to add a large amount of the preservative in order for the preservative used in Comparative Examples to exhibit preservative property, and the addition of a large amount of the preservative results in an imbalance of the nanoemulsion, and therefore the stability of the nanoemulsion decreases. In Comparative Example 6, neither preservative property nor stability was observed. In Comparative Examples 8 through 12, stability was observed but no preservative property was observed. Comparative Examples include the nanoemulsion that does not contain a poorly water-soluble functional component. However, the presence or absence of the poorly water-soluble functional component does not affect the evaluation of preservative property. Therefore, the preservative property can be evaluated regardless of the presence or absence of the poorly water-soluble functional component.

That is, it can be seen that, in a case where parabens, alkanediols, and phenoxyethanol are used as preservatives, it is not possible to obtain a nanoemulsion having stability and preservative property. It can be seen that there are limited preservatives that do not affect the stability of nanoemulsions of small emulsion particle diameter and can exert preservative properties, and the use of organic acids (salts) and biguanide-based compounds is effective.

### [Example 21]

A nanoemulsion was prepared in a manner similar to that in Example 1, except that potassium sorbate was used in place of sodium benzoate as a preservative, PEG-50 hydrogenated castor oil isostearate was used in place of polyoxyethylene (9) oleyl ether and polyoxyethylene (7) lauryl ether as a surfactant, triethylhexanoin was used in place of squalane as an oil, and the weight ratios of the components were changed to the weight ratios shown in Table 5. The obtained nanoemulsion was evaluated. The results are shown in Table 5.

**[Table 5]**

| Classification | Component name | | | Example 21 | Example 22 | Example 23 | Example 24 | Example 25 | Example 26 |
|---|---|---|---|---|---|---|---|---|---|
| pH adjuster | Citric acid-sodium citrate buffer (pH=4.8) | | | 71.00 | 71.00 | | | | |
| | Citric acid-sodium citrate buffer (pH=5.6) | | | | | 70.20 | 70.20 | 70.50 | 71.00 |
| Surfactant | PEG-50 hydrogenated castor oil isostearate | | | 24.00 | 24.00 | 24.00 | 24.00 | 20.00 | 20.00 |
| Oil | Triethylhexanoin | | | 2.50 | | 3.00 | | | |
| | Rice bran oil | | | | 2.50 | | 3.00 | 7.00 | 7.00 |
| Poorly water-soluble functional component | *γ* -oryzanol | | | 0.50 | 0.50 | | | | |
| | Coenzyme Q10 | | | | | 0.80 | 0.80 | | |
| | Resveratrol | | | | | | | 0.50 | |
| Preservative aqueous solution | Sodium benzoate | | | | | 0.30 | | | |
| | Potassium sorbate | | | 0.30 | 0.30 | | 0.30 | 0.30 | 0.30 |
| | Ion-exchanged water | | | 1.70 | 1.70 | 1.70 | 1.70 | 1.70 | 1.70 |
| | pH | | | 5.6 | 5.6 | 5.8 | 5.9 | 5.5 | 5.5 |
| | Particle diameter (nm) | | | 20.54 | 20.16 | 21.61 | 21.34 | 26.54 | 28.12 |
| | Particle size distribution index PDI | | | 0.020 | 0.047 | 0.065 | 0.071 | 0.048 | 0.055 |
| | Subpeaks with average particle diameter of 100 nm or more (%) | | | 0 | 0 | 0 | 0 | 0 | 0 |
| | Surfactant/(oil+poorly water-soluble component) | | | 8.89 | 8.89 | 6.32 | 6.32 | 2.67 | - |
| | LogP of poorly water-soluble component | | | 12 | 12 | 11 | 11 | 2.57 | - |
| | -0.38 × [LogP]+5.169 | | | 0.609 | 0.609 | 0.989 | 0.989 | 4.192 | - |
| | Preservative property test | Method A | After inoculation (pieces/g) | 140,000 | 140,000 | 140,000 | 140,000 | 140,000 | 140,000 |
| | | | After 21 days (pieces/g) | <10 | <10 | 46,000 | 62,000 | <10 | <10 |
| | | | Preservative property result | Excellent | Excellent | Good | Good | Good | Good |
| | | Method B | Preservative property result | Good | Good | Good | Good | Good | Good |
| | Stability after 21 days | | | Good | Good | Good | Good | Good | Good |

### [Example 22]

A nanoemulsion was prepared in a manner similar to that in Example 21, except that a rice bran oil was used in place of triethylhexanoin as an oil and the weight ratios of the components were changed to the weight ratios shown in Table 5. The obtained nanoemulsion was evaluated. The results are shown in Table 5.

### [Example 23]

A nanoemulsion was prepared in a manner similar to that in Example 21, except that sodium benzoate was used in place of potassium sorbate as a preservative, a citric acid-sodium citrate buffer (pH=5.6) was used in place of the citric acid-sodium citrate buffer (pH=4.8) as a pH adjuster, coenzyme Q10 was used in place of γ-oryzanol as a poorly water-soluble functional component, and the weight ratios of the components were changed to the weight ratios shown in Table 5. The obtained nanoemulsion was evaluated. The results are shown in Table 5.

### [Example 24]

A nanoemulsion was prepared in a manner similar to that in Example 23, except that potassium sorbate was used in place of sodium benzoate as a preservative, a rice bran oil was used in place of triethylhexanoin as an oil, and the weight ratios were changed to the weight ratios shown in Table 5. The obtained nanoemulsion was evaluated. The results are shown in Table 5.

### [Example 25]

A nanoemulsion was prepared in a manner similar to that in Example 24, except that potassium sorbate was used as a preservative, resveratrol was used in place of coenzyme Q10 as a poorly water-soluble functional component, and the weight ratios of the components were changed to the weight ratios shown in Table 5. The obtained nanoemulsion was evaluated. The results are shown in Table 5.

### [Example 26]

A nanoemulsion was prepared in a manner similar to that in Example 25, except that no poorly water-soluble functional component was added. The obtained nanoemulsion was evaluated. The results are shown in Table 5.

### [Result 5]

As shown in Table 5, in a case where the organic acid (salt) was used as a preservative, regardless of types of the surfactant, the oil, and the poorly water-soluble functional component, the number of living bacteria on the 21st day in the method A was less than 100% of the number of living bacteria after inoculation, the number of living bacteria on the 28th day was less than 100% of the number of living bacteria after inoculation for at least one of the five types of test bacteria in the method B and, for none of the five types of test bacteria, the number of living bacteria on the 28th day was 100% or more of the number of living bacteria after inoculation. In Example 26, a poorly water-soluble functional component was not contained. However, the presence or absence of the poorly water-soluble functional component does not affect the effect of preservative property. Therefore, the preservative property can be evaluated regardless of the presence or absence of the poorly water-soluble functional component.

Further, in a visual observation of the nanoemulsion on the 21st day, the liquid state was uniform and transparent.

That is, it can be seen that, in a case where at least one selected from the sodium benzoate and the potassium sorbate is contained as an organic acid (salt), the amount of the sodium benzoate or the potassium sorbate is 0.30% by weight, relative to the total weight of the nanoemulsion, and the pH is 6.0 or less, a stable nanoemulsion having preservative efficacy can be obtained.

### Industrial Applicability

The composition for preparing a nanoemulsion and the nanoemulsion in accordance with an embodiment of the present invention include the above-described configuration, and therefore have preservative property and are excellent in stability. Therefore, the composition and the nanoemulsion can be suitably used in the commercialization and selling of nanoemulsions in cosmetic products, pharmaceutical products, foods, industrial fields, and the like.

## Claims

1. A nanoemulsion which contains emulsion particles that have an average particle diameter of 30 nm or less and have a particle size distribution index of 0.14 or less, and which does not contain emulsion particles of 100 nm or more,
- said nanoemulsion comprising a surfactant component, an oil, a poorly water-soluble functional component, an aqueous medium, and a preservative,
- the preservative comprising at least one selected from an organic acid (salt) and a biguanide-based compound.

2. The nanoemulsion as set forth in claim 1, wherein:
- the organic acid (salt) contains at least one selected from sorbic acid and a salt thereof, benzoic acid and a salt thereof, and dehydroacetic acid and a salt thereof; and
- said nanoemulsion further comprises a pH adjuster.

3. The nanoemulsion as set forth in claim 1, wherein:
- the organic acid (salt) contains at least one selected from potassium sorbate, sodium benzoate, and sodium dehydroacetate;
- a contained amount of the potassium sorbate is 0.05% by weight to 0.50% by weight, relative to a total weight of said nanoemulsion;
- a contained amount of the sodium benzoate is 0.10% by weight to 0.80% by weight, relative to the total weight of said nanoemulsion;
- a contained amount of the sodium dehydroacetate is 0.10% by weight to 0.60% by weight, relative to the total weight of said nanoemulsion; and
- a pH of said nanoemulsion is 6.0 or less.

4. The nanoemulsion as set forth in claim 1, wherein:
- the organic acid (salt) contains sodium benzoate in an amount of 0.36% by weight to 0.60% by weight, relative to a total weight of said nanoemulsion.

5. The nanoemulsion as set forth in claim 1, wherein:
- the biguanide-based compound contains polyaminopropyl biguanide.

6. The nanoemulsion as set forth in claim 1, wherein:
- the biguanide-based compound contains polyaminopropyl biguanide in an amount of 0.002% by weight to 1.00% by weight, relative to a total weight of said nanoemulsion.

7. The nanoemulsion as set forth in claim 1, wherein:
- a weight ratio of the surfactant component to the oil is 2.80 or more and 240 or less; and
- a weight ratio of the surfactant component to a total weight of the oil and the poorly water-soluble functional component is 2.60 or more and 200 or less.

8. The nanoemulsion as set forth in claim 1, wherein:
- the surfactant component contains at least one selected from polyoxyalkylene alkyl ether, polyoxyalkylene alkenyl ether, a polyoxyethylene castor oil, an alkylene oxide adduct of sorbitan fatty acid ester, and polyoxyethylene hydrogenated castor oil alkanoate.

9. A pharmaceutical product, a functional food, a cosmetic product, a coating agent, or a pesticide, each comprising a nanoemulsion recited in any one of claims 1 through 8.
